# EUROPÄISCHE PATENTSCHRIFT

(11) **EP 2 622 317 B1**
(45) Veröffentlichungstag und Bekanntmachung des Hinweises auf die Patenterteilung: **06.04.2016**
(21) Anmeldenummer: 11763688.6
(22) Anmeldetag: 29.09.2011
(51) Int. Cl.: G01N 1/28, B26D 1/08, B26D 7/01, B23D 45/00, B26D 7/02, B26D 3/16, A61B 5/00, B26D 1/04, B26D 7/06

(54) **VERFAHREN ZUM SCHNEIDEN VON HAAREN**
METHOD FOR CUTTING HAIR
MÉTHODE POUR COUPER DES CHEVEUX

(30) Priorität: 01.10.2010 DE 102010041881
(43) Veröffentlichungstag der Anmeldung: 07.08.2013
(73) Patentinhaber: Lô, Insa, 71144 Steinenbronn (DE)
(72) Erfinder: Lô, Insa, 71144 Steinenbronn (DE)
(74) Vertreter: Franke, Dirk
(86) Internationale Anmeldenummer: PCT/EP2011/067030
(87) Internationale Veröffentlichungsnummer: WO 2012/041982

(56) Entgegenhaltungen:
- JP-A- 2008 062 370
- US-A- 1 424 472
- US-A1- 2005 258 285
- S. F. STONE ET AL: "Production of hair intercomparison materials for use in population monitoring programmes for mercury and methylmercury exposure", FRESENIUS' JOURNAL OF ANALYTICAL CHEMISTRY, Bd. 152, 1. Januar 1995 (1995-01-01), Seiten 184-187, XP55015679,

## Beschreibung

### Technisches Gebiet

Die vorliegende Erfindung betrifft Verfahren zum Schneiden von Haaren, Fasern oder dünnen, flexiblen Materialien, sowie Verwendungen zur Herstellung von Haarpartikeln definierter Länge zur segmentalen Haaranalyse sowie zur Herstellung von Referenzproben zur Haaranalytik.

### Beschreibung

Die Analyse von Haaren ist beispielsweise in der Forensik, im Zusammenhang mit Untersuchungen auf Substanzmissbrauch (z. B. Nachweis gängiger Drogen wie Cannabinoide, Amphetamine und Benzodiazepine, Detektion von Ethylglucuronid bei Alkoholabusus), bei arbeitsmedizinischen Fragestellungen (z. B. zum Nachweis von Schadstoffexposition) oder im veterinär- und sportmedizinischen Bereich (z. B. zum Nachweis unerlaubter pharmakologischer Stoffe) wegen ihres nur gering invasiven Charakters von wachsender Bedeutung. Der Nachweis unterschiedlichster Stoffe (wie zum Beispiel Drogen, Medikamente und Schadstoffe) in Haaren ist durch die Einlagerung der entsprechenden Stoffe in die Haarsubstanz möglich. Dabei werden die Stoffe zunächst im Blut transportiert und gelangen über den Haarfollikel in das wachsende Haar, welches die Substanz über einen längeren Zeitraum konserviert.

Messungen bilden die Grundlage der meisten Prüfungen, Analysen und quantitativen Vergleiche. Fast immer gilt: Was nicht gemessen werden kann, kann nicht verbessert werden. Messungen ohne Standards sind nicht möglich. Die weitaus meisten Standards sind Referenzmaterialien. Die Zuverlässigkeit und Richtigkeit von Messungen hängt wesentlich von der Verfügbarkeit von Referenzmaterialien ab. Das gilt in besonderem Maße für chemische Messungen. Aus diesem Grund spielen Referenzmaterialien in den Rückführungsstrukturen der chemischen Analytik eine zentrale Rolle.

Referenzmaterialien sind in der Forensischen, Toxikologischen und Klinischen Chemie für flüssige Matrices wie Vollblut, Plasma, Serum oder Urin kommerziell etabliert, während es für die Feststoff-Matrix Haar kaum geeignete und ausreichend standardisierte Referenzmaterialien auf dem Markt gibt. Der Hauptgrund hierfür ist, dass solche Proben bislang nicht kostengünstig und in ausreichend großen Mengen herstellbar sind.

Ein grundsätzliches Problem bei der Herstellung von Haar-Referenzmaterialien besteht in der Realisierung einer ausreichenden Homogenität großer Chargen. Um kommerziell verwertbare Chargengrößen von mindestens 100 bis 1.000 Gramm zu erhalten, müssen Einzelhaarproben mehrerer Haarspender auf Eignung analysiert und zu erheblich größeren Pools vereinigt werden. Die durch Mischung erzielbare Homogenität solcher Feststoffgemische hängt wesentlich von deren Teilchenzahl und ihrer Größenverteilung ab, so dass eine möglichst feine Haarzerkleinerung angestrebt wird. Diese muss umso feiner erfolgen, je ungünstiger das Verhältnis der kleinsten Einzelspende zur Gesamtmenge des Pools ist, damit eine hohe statistische Wahrscheinlichkeit einer ausreichend gleichmäßigen Verteilung gegeben ist. Die Zerkleinerung von Haarproben stellt aus diesem Grund einen entscheidenden Gesichtspunkt bei der Herstellung größerer Haarpools dar.

Bei jeder Haarzerkleinerung durch Schneiden entsteht ein Gemisch von Partikeln in unterschiedlicher und bislang nur sehr eingeschränkt kontrollierbarer Größenverteilung. Feststoffgemische weisen ein größenabhängiges Migrationsverhalten ihrer Partikel auf, das erst bei feinster Vermahlung (Pulver) praktisch keine Rolle mehr spielt. Dieser "Popcorn-Effekt" ist umso größer, je stärker sich die Partikel in ihrer Größe unterscheiden. Dies führt zu einer allmählichen Anreicherung kleinerer Partikel im unteren Bereich und größerer Partikel im oberen Bereich des Gemisches. Mit Homogenitätseinbußen durch Entmischungsvorgänge ist insbesondere während Abfüllprozessen (Dosierung, Portionierung) sowie nach längeren Transport- und Lagerungszeiten abgefüllter Einzelgebinde zu rechnen. Für ein Referenzmaterial ist jedoch Voraussetzung, dass jede Einzelprobe repräsentativ für die Zusammensetzung der ursprünglichen Gesamtcharge ist.

Die Pulverisierung von Haaren durch Mahlen ist das bislang am besten geeignete Verfahren zur Herstellung großer und zugleich homogener Haarpools. Verschiedene Mahlgeräte und -verfahren kommen zum Einsatz, wobei mit allen eine enorme Erhöhung der Teilchenzahl erzielt werden kann. Die Pulverisierung beinhaltet allerdings einige Nachteile: die mechanische Zerstörung der strukturellen Integrität der Haarfaser und die durch das Mahlen erzielte extreme Oberflächenvergrößerung bedingen eine nur begrenzte Vergleichbarkeit der gemahlenen Haar-Referenzprobe mit dem für die Analyse geschnittenen Untersuchungsgut in den präanalytischen Wasch-und Extraktionsschritten. Eine möglicherweise infolge des Mahlvorganges auftretende thermische Schädigung des Mahlgutes hat zusätzliche Auswirkungen auf die Vergleichbarkeit. Da das Untersuchungsgut aber aufgrund der für forensische Untersuchungen geforderten Bestimmungen im geschnittenen Zustand untersucht werden muss, eignet sich das pulverisierte Mahlgut sich nicht mehr zur Überwachung des kompletten analytischen Verfahrens.

Die europäische Patentschrift EP 1 933 985 B1 offenbart eine Kugelmühle, geeignet für eine chargenweise, schnelle Zerkleinerung von mittelharten bis harten Proben auf feinste Partikelgrößen. Diese kleinsten Partikelgrößen führen zwar zu der gewünschten Homogenisation, umgehen also den "Pop-Corn Effekt", besitzen aber, als Referenzmaterial, den Nachteil der infolge des strukturellen Unterschieds nur geringen Vergleichbarkeit mit geschnittenem authenthischen Untersuchungsmaterial. Das Problem der im Verlauf des Mahlvorganges möglicherweise auftretenden thermischen Schädigung kann durch Gefrieren der Proben umgangen werden (Vergleich: Spex Kryomühlen). Die bereits genannten Nachteile, die sich aus den strukturellen Unterschieden von Referenz-und Untersuchungsmaterial ergeben, bleiben allerdings weiterhin bestehen.

Die US-Patentanmeldung US 2005/0258285 A1 offenbart eine Vorrichtung zum Zerkleinern von biologischen Proben mittels rotierender Oberflächen.

Der Fachbeitrag S.F. Stone et al.: "Production of hair intercomparison materials for use in population monitoring programmes for mercury and methyl mercury exposure", Fresenius J. Anal. Chem. 1995, 352, 184-187 (XP55015679) offenbart ein Verfahren zur Herstellung von Haarvergleichsmaterialien zur Verwendung in Quecksilber- und Methylquecksilber-Screening-Verfahren.

Eine Zerkleinerung durch Schneiden der ungemein filigranen und schwer zu handhabenden Haarfasern ist bislang nur manuell, nur für kleinere Mengen und nur mit großen Varianzen in der Schnittlänge der so erhaltenen Haarpartikel zu bewerkstelligen. Beispielsweise existieren Vorrichtungen zum Schneiden von Haarfasern im Stand der Technik, welche in der Regel aus einer Halte- und einer Schneidevorrichtung bestehen. Als beispielhafte Vertreter der Gruppe sind Mikrotome zu nennen, welche zum Schneiden von Einzelhaaren geeignet sind, jedoch nicht dafür verwendet werden können, beispielsweise Referenzmaterial mit ausreichender Homogenität und definierter Größenverteilung schnell und einfach herzustellen. Diese Verfahren beinhalten überdies den Nachteil, dass eine Einbettung des zu schneidenden Materials erforderlich ist, wobei typischerweise Paraffin, Polyethylenglykol, Celloidin, Gelatine, Agar und Kunstharze zum Einsatz kommen. Diese Medien müssen nach dem Schneiden durch einen oder mehrere zusätzlichen Reinigungsschritte entfernt werden, wodurch das Risiko einer Kontamination der Haare durch Einbettmediums-Rückstände erhöht wird.

### Darstellung der Erfindung, Aufgabe, Lösung, Vorteile

Ausgehend von den vorgenannten Verfahren des Standes der Technik liegt der vorliegenden Erfindung deshalb die Aufgabe zugrunde, Verfahren bereitzustellen, mit welchen menschliche oder tierische Haare, Fasern und andere lange, flexible Materialien dicht gebündelt in Abschnitte von definierter Länge mit kontrollierbarer Größenverteilung und bei Erhalt ihrer strukturellen Integrität kontaminationsfrei hergestellt werden können, die also die oben genannten Nachteile des Standes der Technik nicht aufweisen. Die Verfahren sollen dabei kostengünstig arbeiten und für den Routineeinsatz und zur Herstellung insbesondere von Referenzmaterial gut geeignet sein. Die durch die Verfahren hergestellten Abschnitte sollen zudem eine hervorragende Homogenität aufweisen.

Diese der Erfindung zugrunde liegenden Aufgabe wird durch die in den Ansprüchen definierten Verfahren gelöst, wie sich auch aus den beiliegenden Ausführungsbeispielen und experimentellen Daten ergibt.

In einem ersten Aspekt betrifft die vorliegende Erfindung deshalb ein Verfahren gemäß Anspruch 1. In dem Verfahren kann eine Vorrichtung zum Schneiden von Haaren, Fasern oder dünnen, flexiblen Materialien verwendet werden. Eine solche Vorrichtung umfasst mindestens ein Halteelement (2) mit zylindrisch ausgebildetem Innenraum. Das Halteelement (2) ist dabei geeignet, eine zu schneidende Probe (1) aus einer Vielzahl von Haaren, Fasern oder dünnen, flexiblen Materialien in Längsrichtung zu fixieren und gegebenenfalls zu bündeln. Die Vorrichtung umfasst überdies eine Schneidevorrichtung (3). Diese Schneidevorrichtung (3) ist geeignet, die in Längsrichtung fixierte und gegebenenfalls gebündelte Probe im Wesentlichen rechtwinklig (d.h. orthogonal) zur Zylinderachse des Innenraums des Halteelements (2) zu schneiden.

Als Halteelement kann erfahrungsgemäß jedes Halteelement verwendet werden, welches einen zylindrisch ausgebildeten Innenraum umfaßt, der geeignet ist, eine zu schneidende Probe aus einer Vielzahl von Haaren, Fasern oder dünnen flexiblen Materialien aufzunehmen. Der Innenraum ist zylindrisch ausgebildet, d.h. der Innenraum wird durch eine Grund- und eine Deckfläche, sowie durch eine Mantel- bzw. Zylinderfläche, die von parallelen Geraden gebildet wird, begrenzt. Vorzugsweise handelt es sich um einen geraden Zylinder. In einer bevorzugten Ausführungsform besitzt das zylindrisch ausgebildete Halteelement mindestens ein offenes Ende. Das Halteelement kann aus jedem beliebigen Material aufgebaut sein, welches eine Fixierung und gegebenenfalls eine Bündelung der Probe ermöglicht. Unter Fixieren wird erfindungsgemäß die verrutschungsfreie Lagerung der Probe innerhalb des zylindrisch ausgebildeten Innenraums verstanden, wodurch eine Dislokation sowohl der gesamten Probe gegenüber dem Halteelement als auch einzelner Probenbestandteile gegeneinander vor und während des Schneideprozesses verhindert wird. Als Bündeln der Probe bezeichnet man die Ausrichtung der Probe parallel zur Achse des zylindrisch ausgebildeten Innenraums des Halteelementes und/oder die räumlich weitgehend enge Anordnung der Bestandteile der Probe gegeneinander.

Das durch Fixieren und gegebenenfalls Bündeln bedingte Fehlen von Dislokation ist von besonderem Vorteil für die Homogenität der aus dem Schneideprozess resultierenden Abschnitte, da die so fixierte und gegebenenfalls gebündelte Probe infolge des hohen Volumenanteils der Probe und geringer Hohlraumbildung insgesamt eine homogenere Beschaffenheit aufweisen kann als nicht gebündeltes Schnittmaterial. Außerdem kann eine dichte Bündelung weitgehend das Herausziehen einzelner Fasern aus der Probe während des Schneideprozesses verhindern.

Vorzugsweise ist das Halteelement selbst zylindrisch ausgebildet. Beispielsweise kann das Halteelement eine Röhre oder ein Hohlzylinder mit ebenfalls zylindrisch ausgebildetem Innenraum sein. Die Geometrie des zylindrisch ausgebildeten Halteelements, bzw. dessen zylindrisch ausgebildeten Innenraums, betreffend können Grund- und Deckfläche sowie die damit korrespondierende Mantelfläche grundsätzlich aus einer Vielzahl möglicher Formen gebildet sein, vorzugsweise ist der gerade Zylinder im Querschnitt kreisförmig ausgebildet, wobei eine rundzylindrische Form mit rechteckigem Vertikalschnitt entsteht. Die Kreisförmigkeit der Grund- und Deckfläche des Haltelements bzw. des zylindrisch ausgebildeten Innenraums ermöglicht besonders vorteilhaft eine weitgehend gleichmäßige Verteilung des eingebrachten Probenmaterials in den Innenraum. Vorzugsweise besitzt der zylindrisch ausgebildete Innenraum einen Durchmesser von 1 bis 5 mm, bevorzugt 2 bis 4 mm und insbesondere ungefähr 3 mm. Auf diese Weise können im Innenraum beispielsweise 200 bis 800, insbesondere 375 bis 560 nebeneinander liegende Einzelhaare mit durchschnittlichem Längengewicht von 1 mg/15 cm gleichzeitig fixiert und gegebenenfalls gebündelt werden.

Grundsätzlich ist das Halteelement hinsichtlich seiner Länge nicht beschränkt, wobei seine Längenausdehnung vorzugsweise an die physikalischen Eigenschaften der zu schneidenden Probe angepasst ist. Vorteilhafterweise ist das Halteelement röhrenförmig ausgebildet. Unter einem Rohr versteht man einen länglichen Hohlkörper, dessen Länge in der Regel wesentlich größer als sein Querschnitt ist. In einer besonderen Ausführungsform ist das Halteelement ein Schlauch, wenn das Halteelement im Wesentlichen aus flexiblem Material gefertigt ist. Vorzugsweise weist das Halteelement eine Länge von 5 bis 10 cm, insbesondere von circa 8 cm auf.

Das Material des Halteelementes unterliegt grundsätzlich keinen besonderen Beschränkungen. Vorzugsweise wird die Probe entweder unmittelbar vor dem Halteelement geschnitten oder aber dieses in den Schnitt mit einbezogen. Im Besonderen ist das Material des Halteelements an die Beschaffenheit der Probe angepasst. Das Halteelement besteht vorzugsweise aus einem schneidbaren Material. Idealerweise besteht das Halteelement im Wesentlichen aus einem Material, welches aus organischem oder anorganischem Polymer, Metall, Papier, gewickeltem Rohr oder Kombinationen davon ausgewählt ist. Unter Polymer wird ein beliebiges Polymer oder Copolymer verstanden, welches auch einen oder mehrere gebräuchliche Zusatzstoffe enthalten kann. Vorzugsweise besteht das Halteelement aus einem Polymer, welches aus der Gruppe der thermoplastischen oder der elastomeren Polymere oder einer Kombination davon ausgewählt ist, insbesondere aus der der thermoplastischen Polymere. Beispielsweise kann das Halteelement ein thermoplastischer Polymerschlauch sein. Dieser zeichnet sich durch besonders vorteilhafte Materialeigenschaften aus, da er in einem weiten Temperaturbereich flexibel ist, sowie eine hohe mechanische Belastbarkeit, insbesondere eine hohe Verschleiß-, Knick- und Reißfestigkeit besitzt. Vorzugsweise weist das Halteelement elastische Eigenschaften auf und zeigt gleichzeitig ausreichende Festigkeit, um eine Probe zu halten. Infolge seiner Festigkeit ist ein solches Halteelement hervorragend in der Lage, das erfindungsgemäße Probenmaterial ausreichend zu fixieren und gegebenenfalls in erforderlicher Dichte zu bündeln. Vorzugsweise besteht das Halteelement aus einem Polymer, welches aus Polystyrol, Polyvinylchlorid, Polyurethan, Polyamid, Polyethylen, Polypropylen oder Silikon ausgewählt ist, insbesondere aus Polyurethan.

Im Hinblick auf beim Schneideprozess möglicherweise durch Reibungsprozesse entstehende elektrostatische Aufladungen, die eine Trennung von Probe und Halteelement nach dem Abschneiden erschweren können, ist ein Halteelement aus einem Polymer mit möglichst geringem Oberflächenwiderstand und/oder geringem Ableitwiderstand bevorzugt. Unter Oberflächenwiderstand versteht man den elektrischen Widerstand, welcher auf der Oberfläche eines Gegenstandes unter definierten Bedingungen gemessen wird. Polymeroberflächen können sich ab einem Oberflächenwiderstand von 10¹⁰ Ω elektrostatisch aufladen. Ein Gegenstand wird als ableitfähig bezeichnet, wenn der Oberflächenwiderstand zwischen circa 10⁴ Ω und 10¹¹ Ω, gemessen bei 23 °C und 30 % relativer Luftfeuchte, oder zwischen circa 10⁴ Ω und 10⁹ Ω, gemessen bei 23 °C und 50 % relativer Luftfeuchte, beträgt. Das Halteelement besteht vorzugsweise aus einem Polymer mit einem Oberflächenwiderstand von maximal 10¹⁰ Ω, insbesondere maximal 10⁸ Ω. Wenn das Halteelement aus Papier oder aus Polymer besteht, dann ist es bevorzugt, dass dieses ganzflächig oder zumindest teilflächig mit einer antistatisch wirksamen Schicht beschichtet ist. Hierdurch wird die elektrische Aufladung während des Schneidens verringert. Beispielsweise kann das Haltelement ganzflächig oder teilflächig mit einer Schicht aus einem leitenden Antistatikum (z.B. aufgedampftes Metall, wie beispielsweise Kupfer, Gold oder Silber) oder nicht-leitenden Antistatikum (z.B. einem aufgetragenen, quaternären Ammoniumsalz, wie beispielsweise einem Polyquaternium, Behentrimoniumchlorid oder Cocamidopropylbetain, einem aufgetragenen aliphatischen, wahlweise ethoxylierten Amin oder Amid, einem Phosphorsäureester, eine Polyethylenglycolester oder einem Polyol) beschichtet sein. Alternativ oder zusätzlich kann das Halteelement auch eine intrinsische Ableitfähigkeit aufweisen. Ein solches Halteelement kann besonders vorteilhaft in der Vorrichtung eingesetzt werden, da es keine Kontaminationsquelle für die Probe darstellt. Dabei ist der durch Abrieb entstehende auch bei intrinsischen Antistatika beobachtete Verlust der antistatischen Wirkung in der Vorrichtung zu vernachlässigen, da individuelle aus Polymer gebildete Halteelemente nach dem Schneideprozess nicht wiederholt verwendet werden müssen. Beispielsweise kann das Halteelement aus einem Polymer, wie beispielsweise Polyurethan bestehen, in welchem intrinsisch ein quaternäres Ammoniumsalz, wie beispielsweise ein Polyquaternium, Behentrimoniumchlorid oder Cocamidopropylbetain, ein aliphatisches, wahlweise ethoxyliertes Amin oder Amid, ein Phosphorsäureester, ein Polyethylenglycolester oder ein Polyol enthalten ist. Besonders vorteilhaft ist es, wenn das Halteelement aus einem durchsichtigen oder durchscheinenden Polymer besteht, in welchem Indiumzinnoxid als intrinsisches Antistatikum eingebettet ist. Indiumzinnoxid bietet den Vorteil, dass es selbst durchsichtig ist, so dass die Probe innerhalb des Halteelements von außen gesehen werden kann.

In einer alternativen Ausführungsform kann das Halteelement aus einem leitfähigen Polymer, beispielsweise PEDOT:PSS oder einer leitfähigen Polymer-Nanofaser, insbesondere Polyanilin-Nanofaser aufgebaut sein. Ein Halteelement aus diesen Materialien zeigt hervorragende antistatische Wirkung.

Vorzugsweise ist das Halteelement jedoch aus Polyurethan aufgebaut. Solche Halteelemente weisen einen günstigen Kompromiss der mechanischen Parameter Elastizität, Oberflächenrauheit und Druckfestigkeit auf, wodurch eine hervorragende Fixierung bei gegebenenfalls ausreichend dichter Bündelung der Probe erreicht wird. Unter Elastizität (Einheit z. B.: Elongation in %, ASTM Test D-412) wird die Verformbarkeit eines Polymers unter Zug- und Druckbelastung verstanden. Unter Oberflächenrauheit versteht man die Beschaffenheit der Polymeroberfläche (Einheit: µm), die den Reibungskoeffizienten µ (Einheit: dimensionslos) bedingt. Im Allgemeinen weisen weichere Materialien einen höheren und härtere Materialien einen niedrigeren Reibungskoeffizienten auf (entsprechend einem trockenen Reibungskoeffizienten µ = 0,3 - 0,4 für weiche Materialien (< 85° Shore A Härte) und µ = 0,15-0,25 für harte Materialien (>85° Shore A Härte). Unter Druckfestigkeit (Einheit z. B.: Nmm²) wird die Widerstandsfähigkeit eines Polymers bei der Einwirkung von axial gerichteten Druckkräften bezeichnet. Bei einer die Druckfestigkeit des Polymers übersteigenden Druckspannung reißt das Polymer. Diese vorteilhaften Materialeigenschaften können durch die geometrischen Parameter des Halteelements beeinflusst werden. In der Regel ist bei höherer Wandstärke eine größere Kraft pro Fläche (N/m²) für das spanfreie Abtrennen der Abschnitte erforderlich, welches einem Auseinanderdrücken des Werkstoffes entspricht. Je größer die für das Durchtrennen notwendige Kraft, desto höher ist in der Regel auch die Beanspruchung der Schneidevorrichtung. Insbesondere Halteelemente aus Polyurethan mit einer Wandstärke von circa 1 mm zeigen die vorteilhafte Kombination aus ausreichender Elastizität und Oberflächenrauheit, welche vor dem Schneiden eine hinreichende Fixierung und gegebenenfalls Bündelung der Probe und nach dem Schneiden eine rasche Trennbarkeit von Abschnitten des Halteelements und der Probe ermöglichen, und ausreichender Druckfestigkeit, die weitgehend einer beim Schneiden auftretende Verformung des Halteelements durch Quetschung entgegenwirkt, das Heraustreten der Probe beim Schneidevorgang verhindert und die Schneidevorrichtung möglichst gering verschleißt.

In einer weiteren bevorzugten Implementierung ist das Halteelement aus Papier ausgebildet. Zur Herabsetzung seines im trockenen Zustand hohen Oberflächenwiderstandes (>10¹⁴ Ω) kann dieses in einer bevorzugten Ausführung mit einer antistatischen Beschichtung versehen werden, um die Ableitfähigkeit zu erhöhen. Als antistatische Beschichtung kann eine wie oben beschriebene Beschichtung verwendet werden. Halteelemente aus Papier mit hoher Glätte des Materials (gemessen nach im Luftstromverfahren nach Bekk, Parker Print Surf oder Bendtsen) bieten zudem vorteilhafte Eigenschaften bei der auf den Schneidevorgang folgenden Trennung der Abschnitte der Probe und des Halteelements.

In einer bevorzugten Implementierung kann das Halteelement mit Hilfe einer steuer- und kalibrierbaren Antriebseinrichtung bewegt werden. Insbesondere kann das Halteelement an einer Führungsvorrichtung befestigt und mittels einer steuerbaren Antriebseinrichtung bewegt werden. Unter Steuerbarkeit der Bewegung versteht man eine durch geeignete Stellsignale in einem bestimmten Zeitraum vermittelte Bewegung in einer definierten Raumrichtung um einen vom Benutzer vorgewählten Längenwert (Vorschub). Unter der definierten Raumrichtung wird eine erste Raumrichtung verstanden. Eine bevorzugte Antriebseinrichtung kann eine Zuführung der zu schneidenden Probe mittels der Führungsvorrichtung im Stop&Go-Betrieb bereitstellen, wobei zum Schneiden das Halteelement und/oder eine Probe angehalten und anschließend um den definierten Vorschub vorbewegt werden kann.

Antriebseinrichtungen sind im Stand der Technik bekannt und beschrieben. In der Vorrichtung unterliegt die Art der Antriebseinrichtung grundsätzlich keinen Beschränkungen. Idealerweise erfolgt der Antrieb motorisch oder manuell, vorzugsweise erfolgt der Antrieb motorisch, insbesondere elektrisch. Eine solche Antriebseinrichtung hat den Vorteil, dass ein oder besonders mehrere Halteelemente während des Schneideprozesses um den vorgewählten Längenwert in einer ersten Raumrichtung bewegt werden kann/können, wobei eine erneute manuelle Einstellung des Vorschubs möglich, aber nicht erforderlich ist. Dies ist besonders vorteilhaft für das Schneiden großer Mengen an Proben, welche beispielsweise zur Herstellung von Referenzhaarproben wünschenswert ist. Der für den Schneideprozess vorteilhafte Stop&Go-Betrieb kann durch einen Leser vermittelt werden, der vom Benutzer definierte Steuerungsmerkmale, wie z. B. die Vorschublänge, erkennt und die Antriebseinrichtung steuert.

Die Schneidevorrichtung kann eine vollständige Trennung der Abschnitte von der Ausgangsprobe ermöglichen. Als Schneidevorrichtung kann eine Säge oder ein Messer verwendet werden. Im Besonderen kann eine spanfreie Zerteilung in Form eines Keilschneideprozesses erfolgen, wobei eine oder zwei Schneiden verwendet werden können. Idealerweise wird die Schneidevorrichtung aus einer in Führungsschienen liegenden starren Messerklinge gebildet, die ein eine Schneidekante tragendes vorderes und ein hinteres Ende aufweist. Die Schneidekante ist dabei ausgewählt aus einer geraden, gebogenen, gewinkelten oder durchbrochenen Form. In einer bevorzugten Implementierung ist die Schneidevorrichtung ein Messer mit gerader oder gewinkelter Schneidekante, welche plankonkav, keilförmig oder hobelförmig geschliffen sein kann. Besonders vorteilhaft ist ein hobelförmiger Schliff des Messers. Insbesondere ist es vorteilhaft, wenn nur die dem Abschnitt zugewandte Seite des Messers angeschliffen ist, während die der geschnittenen Probe zugewandte Seite des Messers in vertikaler Richtung flach ausgebildet ist. Der vordere Schliffwinkel von circa 45° kann die Stabilität des Messers erhöhen und eine Verwendung auch bei härterer Materialzusammensetzung des Halteelements und/oder der Probe ermöglichen. Das Messer besteht vorzugsweise aus einem Material, welches aus Stahl, Hartmetall, oder Glas ausgewählt ist, insbesondere besteht es aus gehärtetem Stahl.

Die Schneidevorrichtung ist vorzugsweise zwischen einer ersten und zweiten Stellung nach Art eines Fall- oder Schlagmessers (d.h. Guillotine) zum Abschneiden der Probe beweglich. In einer bevorzugten Implementierung kann die Schneidevorrichtung mit Hilfe einer steuerbaren Antriebsvorrichtung zwischen einer ersten Position (Ruhestellung) und einer zweiten Position (Betriebsstellung) hin und her bewegt werden. Vorzugsweise weist das Messer eine Schneidkante auf, welche sowohl in der ersten wie auch in der zweiten Position im Wesentlichen rechtwinklig zur Längsachse des Halteelements angeordnet ist. Die Raumrichtung der Schneidebewegung liegt dabei im Wesentlichen rechtwinklig zur Längsachse des Halteelements, wobei die Schneidevorrichtung sich aus jeder Richtung dem Halteelement nähern kann, vorzugsweise wird eine vertikale Bewegung ausgeführt. Bevorzugt werden das Halteelement und/oder die Probe für den Schneideprozess in einer Führungsöffnung stabilisiert, wobei die Schneidbewegung im Wesentlichen dicht benachbart zur Führungsöffnung ausgeführt wird.

Das Verfahren umfasst dabei konsekutiv die in Anspruch 1 definierten Schritte.

Vorzugsweise werden im Schritt (A) etwa 200 bis 800 Einzelhaare, insbesondere 375 bis 560 Einzelhaare, mit einem durchschnittlichen Längengewicht von 1 mg/15 cm gleichzeitig eingebracht, ausgerichtet und fixiert. Für die Herstellung beispielsweise von Referenzhaarproben können die Einzelhaare vorzugsweise vor dem Einbringen in der Mitte geknickt werden, so dass für eine Befüllung nur circa 100 bis 400, vorzugweise 190 bis 280 Einzelhaare verwendet werden. Dabei kann das jeweilige Einzelhaar in einem Schritt (B) mindestens doppelt geschnitten werden und der die Knickung enthaltene Abschnitt verworfen werden. Bei gleicher Ausgangsmenge von Spenderhaar wird der Volumenanteil des Schnittmaterials pro Halteelement dementsprechend verdoppelt, wodurch sich die Homogenität der Probe infolge der Bündelung erhöht. Dementsprechend stellt eine Füllung mit in der Mitte geknicktem Haar einen besonderen Vorteil dar, da die Verfügbarkeit von Spenderhaaren zur Gewinnung von Referenzmaterialien eingeschränkt ist.

In einer bevorzugten Implementierung wird der Schritt (B) mindestens zweimal hintereinander durchgeführt.

In einer weiteren bevorzugten Implementierung umfasst das Verfahren einen dritten Schritt (Schritt (C)), welcher nach dem Schritt (B) durchgeführt wird. Der Schritt (C) umfasst das kontaminationsfreie Auffangen des Schnittpräparats.

In einer bevorzugten Implementierung werden die Schritte (B) und (C) jeweils mindestens zweimal hintereinander alternierend durchgeführt.

In einer bevorzugten Implementierung umfasst das Verfahren einen vierten Schritt (Schritt (D)), welcher nach dem Schritt (C) durchgeführt wird. Der Schritt (D) umfasst das Trennen des Abschnitts des Halteelements und der Abschnitte von Haaren, Fasern oder dünnen, flexiblen Materialien unter Herstellung von Schnittpartikeln definierter Länge. Dabei können die Abschnitte des Halteelements in einem Sieb mit einer Maschengröße, welche geringer ist als der Querschnitt des Halteelements und im Wesentlichen größer als der Querschnitt einzelner Probenbestandteile, zurückgehalten werden. Gleichzeitig können die Abschnitte des Probenmaterials durch das Sieb in einer Auffangeinrichtung kontaminationsfrei aufgefangen werden.

In einer bevorzugten Implementierung der vorliegenden Erfindung werden die Schritt (B), (C), und (D) in dieser Reihenfolge nacheinander mindestens zweimal durchgeführt.

Das erfindungsgemäße Verfahren kann zur Herstellung eines Schnittpräparats, umfassend eine Vielzahl von Abschnitten von Haaren, Fasern oder dünnen, flexiblen Materialien und einem Abschnitt des Halteelements verwendet werden. Die Vielzahl von Abschnitten von Haaren, Fasern oder dünnen, flexiblen Materialien nach dem Schritt (B) bzw. während des Schritts (C) sind lösbar in dem Abschnitt des Halteelements fixiert.

In einer bevorzugten Implementierung weist das Schnittpräparat zwischen den Schnittflächen eine Länge von 0,1 bis 5 mm auf, im Besonderen eine Länge von 0,2 bis 4mm, insbesondere eine Länge von 0,5 bis 3mm. So erzeugte Abschnitte von homogener Länge eigenen sich besonders vorteilhaft zur Herstellung von Haarreferenzmaterialien, da ihre strukturellen Eigenschaften denen authentischer Haare aus Gründen der Vergleichbarkeit möglichst nahe kommen müssen. Die Erhaltung der strukturellen Integrität der Haare ist ein besonderer Vorteil gegenüber den aus dem Stand der Technik bekannten Mahlverfahren zur Herstellung von Haarreferenzproben, da die Produkte dieses Verfahrens zwar eine homogene Partikelgröße aufweisen, aber ihre die strukturellen Differenzierung des Haarabschnittes, bestehend aus Cuticula, Cortex, und Medulla (Matrix), komplett zerstört ist. Insbesondere mit denen in Forensik und Toxikologie üblicherweise verwendeten manuell geschnittenen Haarproben besitzen diese Referenzmaterialien nur eine geringe Vergleichbarkeit.

In einem zweiten Aspekt betrifft die vorliegende Erfindung ein Verfahren zum Schneiden von Haaren, Fasern oder dünnen, flexiblen Materialien gemäß Anspruch 5.

In einem dritten Aspekt betrifft die vorliegende Erfindung die Verwendung der erfindungsgemäßen Verfahren zur Herstellung von Haarpartikeln definierter Länge zur segmentalen Haaranalyse.

In einem vierten Aspekt betrifft die vorliegende Erfindung die Verwendung der erfindungsgemäßen Verfahren zur Herstellung von Referenzproben zur Haaranalytik.

### Kurze Beschreibung der Figuren

Im Folgenden werden beispielhaft und nicht abschließend einige besondere Ausführungsformen der Erfindung unter Bezugnahme auf die beiliegenden Figuren beschrieben.

Die besonderen Ausführungsformen dienen nur zur Erläuterung des allgemeinen erfinderischen Gedankens, jedoch beschränken sie die Erfindung nicht.

In den besonderen Ausführungsformen zeigen:
Fig. 1 eine schematische Ansicht einerVorrichtung, die in den erfindungsgemäßen Verfahren verwendet werden kann.
Fig. 2 eine Teilansicht auf ein Halteelement (2) der Vorrichtung, die in den erfindungsgemäßen Verfahren verwendet werden kann.
Fig. 3 eine Draufsicht auf eine Ausführungsform der Vorrichtung, die in den erfindungsgemäßen Verfahren verwendet werden kann.
Fig. 4 eine Draufsicht auf Schnittpräparate, die mit den erfindungsgemäßen Verfahren hergestellt werden können.
Fig. 5 eine Ausführungsform des erfindungsgemäßen Verfahrens im schematischen Ablauf.

### Bevorzugte Ausführung der Erfindung

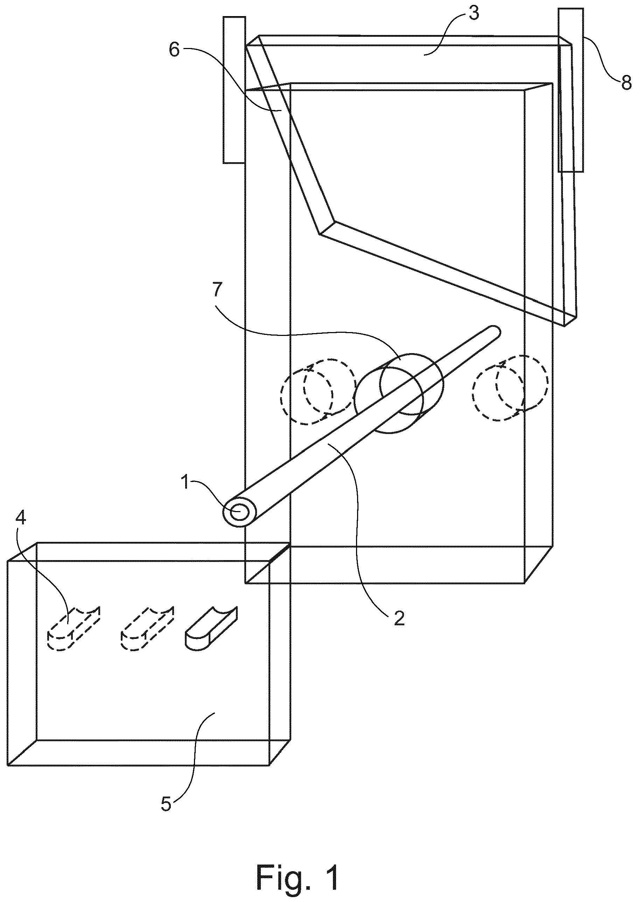

Fig. 1 zeigt eine schematische Ansicht einer Ausführungsform einer Vorrichtung, die in den erfindungsgemäßen Verfahren verwendet werden kann. Die Vorrichtung umfasst mindestens ein Halteelement (2) mit zylindrisch ausgebildetem Innenraum, das geeignet ist, eine zu schneidende Probe (1), die aus einer Vielzahl von Haaren, Fasern oder dünnen, flexiblen Materialien besteht, in Längsrichtung des Halteelements (2) zu fixieren und gegebenenfalls zu bündeln. Die Vorrichtung umfasst ferner mindestens eine Führungsschiene (4), welche an einem beweglichem Element (5) angebracht ist, und welche geeignet ist, das Halteelement in mindestens einer in einer weitgehend senkrecht zum Halteelement stehenden Platte (6) befindlichen Führungsöffnung (7), auszurichten. Die Vorrichtung umfasst weiterhin eine Schneidevorrichtung (3), die geeignet ist, die in Längsrichtung des Halteelements gebündelten und fixierten Fasern oder dünnen, flexiblen Materialien im Wesentlichen rechtwinklig zur Zylinderachse des Innenraums des Halteelements (2) zu schneiden. Die bewegliche Platte kann mit Hilfe eines Motors um ein vom Benutzer definiertes Maß, d.h. den Vorschub (V), in Richtung der Schneidevorrichtung bewegt werden. Der Betrieb der Vorrichtung erfolgt im Stop&Go-Betrieb, da zum Erreichen einer ausreichend hohen Schnittgenauigkeit das Schneiden bei Stillstand (V=0) von Halteelement (2) und Probe (1) erforderlich ist. Das Anhalten des Halteelements (2) wird bei Erreichen des Vorschubwertes V durch einen Leser, in diesem Fall eine Lichtschanke, gesteuert. Der Leser befindet sich hier im messerseitigen Abschnitt der Vorrichtung.

Da eine beliebige Anzahl von Halteelementen (2) verwendet werden kann, kann die Vorrichtung in diesem Ausführungsbeispiel zur wirtschaftlichen Herstellung der ausreichend großer (= kommerziell verwertbarer) Referenz-Haarpools von ausreichender Homogenität verwendet werden, so dass auch kleine Portionierungen des Haarpools, zum Beispiel im Bereich der für toxikologische Untersuchungen verwendeten Mengen von 50 mg bis 100 mg, noch ausreichend repräsentativ für die Zusammensetzung der Gesamtcharge sind. Dabei wird das Haarmaterial nicht, wie im Alternativverfahren, durch Mahlen zerstört, so dass dessen strukturelle Integrität erhalten bleibt. Dies sind zentrale Voraussetzungen, um ein derartiges Material als sogenanntes Referenzmaterial für Haaranalysen jedweder Art einsetzen zu können.

Durch das Schneiden mit Hilfe der Vorrichtung werden Haarabschnitte in kontrollierbarer Größenverteilung erhalten. So gewonnene Einzelfraktionen unterschiedlicher Haarspender können zu homogenen Pools gemischt und ohne Gefahr von Entmischungsvorgängen, die durch starke Größenunterschiede der Einzelabschnitte bedingt sind, in kleine Portionen zur Verwendung als Referenzmaterial geteilt werden. Das auf diese Weise erhaltene Referenzmaterial kann zum Beispiel für forensische Zwecke mit im Stand der Technik bekannten Messmethoden, wie beispielsweise über Gaschromatographie - Massenspektrometrie (GC/MS), Gaschromatographie-Tandem-Massenspektrometrie (GC/MS/MS) oder Flüssigkeitschromatographie-Tandem-Massenspektrometrie (LC/MS/MS) analysiert werden. Aufgrund der hohen Homogenität der Proben sind die Analyseergebnisse sehr gut reproduzierbar.

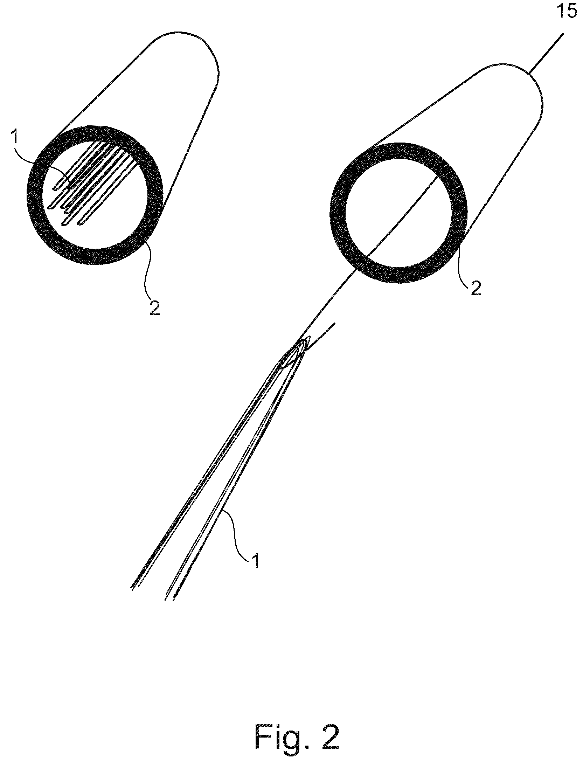

Fig. 2 zeigt eine Teilansicht auf ein Halteelement (2) der Vorrichtung, die in den erfindungsgemäßen Verfahren verwendet werden kann. Die Probe (1), beispielsweise aus Haaren, kann in das Halteelement (2) eingezogen werden, so dass die Haare fest gebündelt nebeneinander liegen. Während des Einziehens, beispielsweise mittels einer Öse (15), werden die Haare vorteilhafterweise gekämmt, so dass ein longitudinales Verrutschen der Probe nicht mehr möglich ist. Das Halteelement (2) kann ein Schlauch oder ein Rohr aus anorganischem oder organischem Kunststoff, Metall, Papier oder gewickeltem Rohr sein. Alternativ kann das Halteelement (2) auch eine Hülse aus diesen Materialien sein. Das Halteelement kann eine beliebige Form aufweisen, wie beispielsweise eine runde, flache oder auch eckige Form.

In diesem Beispiel ist das Halteelement (2) eine Schlauchpatrone mit einer Länge von 8 cm. Mit Hilfe der Schlauchpatrone können 0,2 g bis 0,3 g Haare aufgeladen (d.h. fixiert und gegebenenfalls gebündelt) werden. Bei 1 mg Gewicht pro 15 cm langem Haar (entsprechend dem Durchschnittswert für europäisches Haar) entspricht diese Ladung einer Schlauchpatrone einer Anzahl von 375 bis 560 nebeneinander liegenden Einzelhaaren pro Schlauch. Da die Haarsträhnen bei Einzug in den Schlauch in der Mitte zusammenklappen, was zu einer Verdopplung der im Schlauch nebeneinander liegenden Einzelhaare führt, bedeutet dies, dass ein Schlauch mit einer ca. 17 bis 18 cm langen Haarsträhne, bestehend aus ca. 190 bis 280 Einzelhaaren, befüllt werden kann. Infolge der Verdoppelung kann besonders vorteilhaft und kostengünstig mit weniger Ausgangsmaterial pro Haarspender gearbeitet werden, da durch Fixierung und Bündelung eine größere Packungsdichte der gedoppelten Haarprobe im Halteelement bedingt wird.

In einer bevorzugten Ausführungsform weist das Halteelement einen Innendurchmesser von 3 mm und einen Außendurchmesser von 4 mm auf und besteht aus Polyurethan (PUR, PU), insbesondere aus transparentem Material. Dies ermöglicht es, dass die Probe innerhalb des Halteelements von außen gesehen werden kann. Das Material des Halteelements ist wärmebeständig bis circa 60 oder 70 °C und ist zudem teilweise säure- und/oder ölfest. Die Wandstärke liegt bei 0,5 bis 1 mm. Als Halteelement kann beispielsweise ein Schlauch der Firma Rexroth TU1-8-PUR-4x0.75 verwendet werden. Alternativ oder gleichzeitig kann das Halteelement mit einer antistatisch wirksamen Innenschicht versehen oder aus antistatisch wirksamen Kunststoff gefertigt sein, um einer während des Schneideprozesses auftretenden elektrischen Aufladung von Probe und Halteelement entgegenzuwirken. Um Abschnitte weitgehend frei von Kontamination zu erhalten, können Kunststoffschläuche mit permanenter antistatischer Wirkung eingesetzt werden.

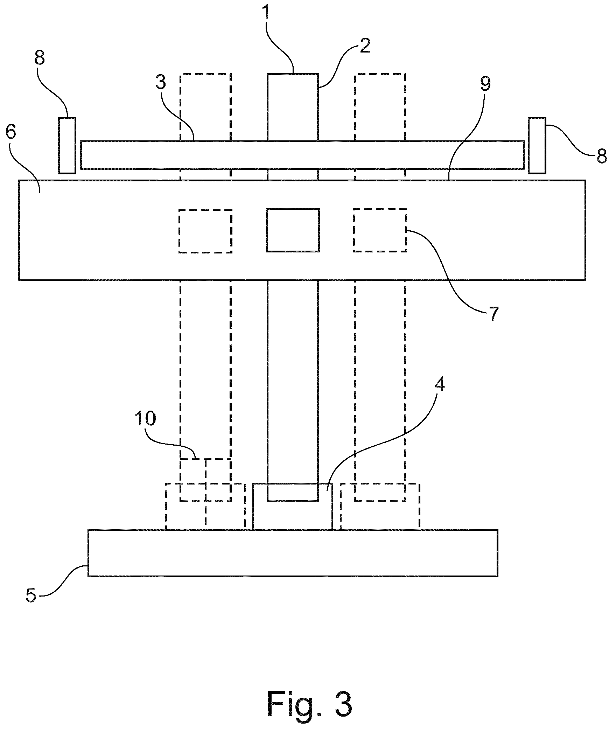

Fig. 3 zeigt eine Draufsicht auf eine Ausführungsform der Vorrichtung, die in den erfindungsgemäßen Verfahren verwendet werden kann. Zwei zusätzliche Halteelemente sind gestrichelt dargestellt, wobei die Anzahl der Halteelemente grundsätzlich keinen Beschränkungen unterliegt. Die Vorrichtung ist geeignet zum Schneiden von Haaren für forensische und toxikologische Zwecke. Innerhalb der Vorrichtung werden die Haare mit einer Schneidevorrichtung (3), bestehend aus einem Messer, einer Säge oder einer Schleifscheibe in der Länge von 0,2 bis 4 mm, insbesondere in einer Länge von 0,5 bis 3 mm zusammen mit dem Haltemittel getrennt. Bevorzugt wird ein in Führungsschienen (8) liegendes Messer eingesetzt, das sich in einer Ruhe- und einer Betriebsstellung befinden kann. In einer bevorzugten Ausführungsform sind die Führungsschienen parallel zur Platte in horizontaler Richtung beweglich und einstellbar, wodurch die Position der Scheidevorrichtung relativ zur Position der in ihrer Anzahl ebenfalls grundsätzlich nicht beschränkten Führungsöffnungen (7) verschoben werden kann. Die im Verlauf des Schneidens positionsabhängig unterschiedlich stark mechanisch beanspruchte Messerschneidkante kann so verschoben werden, wobei der Verschleiß gleichmäßig auf die gesamte Schneidekante verteilt werden und demzufolge die Arbeitszeit des Messers verlängert werden und seine Standzeit verkürzt werden kann. Die Schneidebewegung erfolgt im Wesentlichen rechtwinklig und vertikal zur Achse des Innenraums des Halteelements. Das Messer ist in diesem Beispiel hobeiförmig geschliffen mit gewinkelter Messerkante und befindet sich mit seiner planen Seite im definierten Abstand (9) d von der Platte (6), welche Führungsöffnungen (7) trägt. Der Abstand (9) d beträgt hier maximal 1 mm. Die gewinkelte Ausführung der Schneidekante des Messers dient dazu, eine ziehende Bewegung bei grundsätzlich vertikal gerichteter Schneidebewegung (nach Art einer Guillotine) zu ermöglichen, wodurch insgesamt eine geringere mechanische Beanspruchung der Messerschneidekante und eine glattere Schnittkante des Abschnitts resultiert.

In einem anderen Beispiel werden die Haare mit einem Stößel (10) aus einem als Halteelement dienenden Rohr geschoben und vor dem Rohr getrennt.

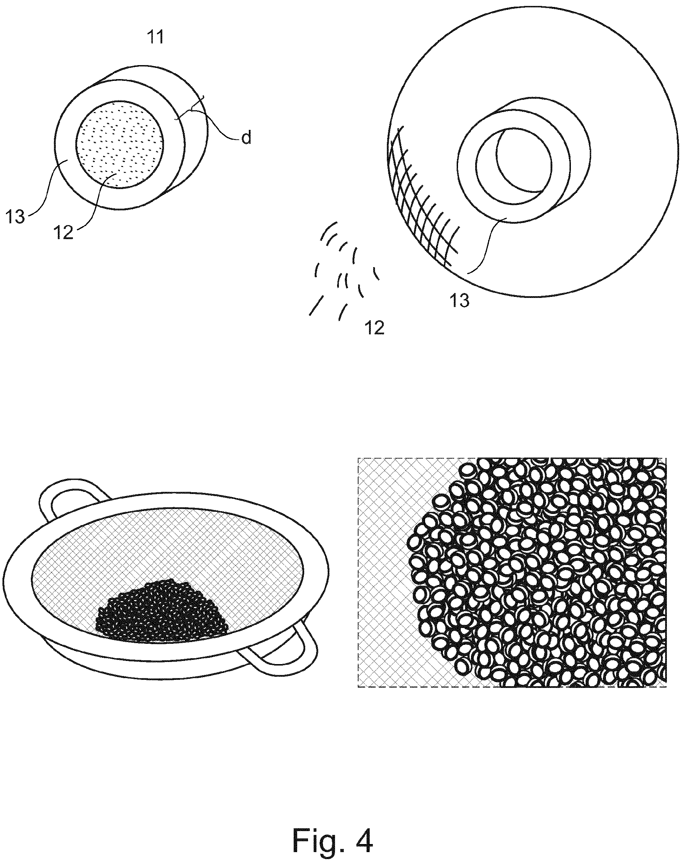

Fig. 4 zeigt eine Draufsicht auf mehrere Schnittpräparate (11), die mit den erfindungsgemäßen Verfahren hergestellt werden können. Die Schnittpräparate bestehen in diesem Fall aus Schlauchringen und geschnittenen Haaren, welche durch nachfolgende Verfahren, wie zum Beispiel Sieben, in einzelnen Bestandteile, die Abschnitte der Proben (12) und des Haltelements (13), getrennt werden können. Die Elastizität eines aus Polymer gebildeten Halteelements beeinflusst wesentlich die Trennbarkeit von Probe und Halteelement, insbesondere Abschnitte mit relativ niedriger Elastizität ermöglichen eine rasche und weitgehend kontaminationsfreie Trennung der Abschnitte ("Herausfallen"). Die Vollständigkeit der Trennung von Halteelement- und Probenabschnitt wird durch eine geringe Retention der Probe an das Halteelement begünstigt. Die Retention kann vermindert werden durch antistatische Eigenschaften des Halteelements, und durch geringe Oberflächenrauheit.

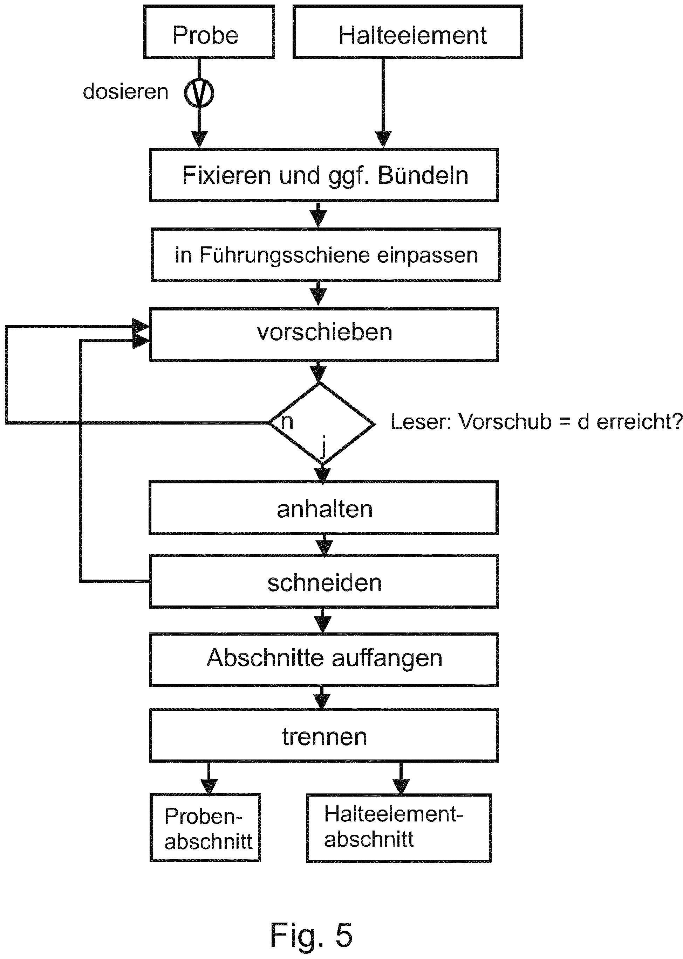

Fig. 5 beschreibt schematisch eine mögliche Ausführungsform des Verfahrens unter Verwendung eines einzelnen mit Probe befüllten Halteelements. Dabei wird eine Probe (1) aus einer Vielzahl von Haaren, Faser oder anderen dünnen, flexiblen Materialien dosiert und in einem Halteelement (2) fixiert und gegebenenfalls gebündelt. Nach Einbringen in eine Führungsschiene (4) wird das Halteelement mittels einer beweglichen Platte (5) in Richtung auf eine feststehende Platte (6) bewegt, welche mit einer Führungsöffnung (7) versehen ist. Die Bewegung erfolgt durch die Führungsöffnung um den vom Benutzer gewählten Vorschub, V. Der Vorschub erfolgt in diesem Ausführungsbeispiel motorisch und kann mittels eines messerseitig angebrachten Lesers, zum Beispiel einer Lichtschranke, gesteuert werden. Bei Erreichen des eingestellten Vorschubs kommt die bewegliche Platte (5) zum Stillstand, entsprechend einem Stop&Go-Betrieb der Vorrichtung, und die Schneidevorrichtung (3) führt einen Schneidevorgang aus. Der aus Probe (1) und Halteelement (2) bestehende Abschnitt (11) wird in einer geeigneten Vorrichtung, zum Beispiel einem Sieb, saufgefangen (Schritt C). Danach kann durch ein geeignetes Verfahren, z. B. durch ein mechanisch Trennverfahren, in die entsprechenden Abschnitte der Proben (12) und des Halteelementes (13), getrennt werden (Schritt D).

### Bezugszeichenliste

- **1**: Probe aus Haaren, Fasern oder anderen dünnen, flexiblen Materialien
- **2**: Halteelement
- **3**: Schneidevorrichtung
- **4**: Führungsschiene für Haltelement
- **5**: bewegliche Platte
- **6**: feststehende Platte
- **7**: Führungsöffnung
- **8**: bewegliche Führungsschiene für Schneidevorrichtung
- **9**: Abstand d
- **10**: Stößel
- **11**: Abschnitt
- **12**: Abschnitt der Probe bestehend aus einer Vielzahl von Haarabschnitten
- **13**: Abschnitt des Halteelements
- **14**: fixiertes Probenende
- **15**: Öse

## Patentansprüche

1. Verfahren zum Schneiden von Haaren, Fasern oder dünnen, flexiblen Materialien umfassend die folgenden konsekutiven Schritte:
(A) Einbringen, Ausrichten und Bündeln einer Probe (1), umfassend eine Vielzahl von Haaren, Fasern oder dünnen, flexiblen Materialien in einem zylindrisch ausgebildeten Innenraum mindestens eines Halteelements (2), und
(B) Schneiden der Probe (1) und des Halteelements (2) mit einer Schneidevorrichtung (3) zur Herstellung eines Schnittpräparats (11), wobei das Schnittpräparat eine Vielzahl von Abschnitten von Haaren, Fasern (12) oder dünnen, flexiblen Materialien und einen Abschnitt des Halteelements (13) umfasst.

2. Verfahren gemäß Anspruch 1, zusätzlich umfassend folgenden weiteren Schritt nach dem Schritt (B):
(C) kontaminationsfreies Auffangen des Schnittpräparats (11).

3. Verfahren gemäß Anspruch 2, zusätzlich umfassend folgenden weiteren Schritt nach dem Schritt (C):
(D) Trennen des Abschnitts des Halteelementes (13) und der Abschnitte von Haaren, Fasern oder dünnen, flexiblen Materialien (12) unter Herstellung von Schnittpartikeln definierter Länge.

4. Verfahren gemäß einem der Ansprüche 1 bis 3 zur Herstellung eines Schnittpräparats (11), umfassend eine Vielzahl von Abschnitten von Haaren, Fasern oder dünnen, flexiblen Materialien (12) und einem Abschnitt des Halteelementes (13), wobei die Vielzahl von Abschnitten von Haaren, Fasern oder dünnen, flexiblen Materialien nach dem Schritt (B) bzw. während des Schritts (C) lösbar in dem Abschnitt des Halteelementes (13) fixiert bleiben.

5. Verfahren zum Schneiden von Haaren, Fasern oder dünnen, flexiblen Materialien umfassend die folgenden konsekutiven Schritte:
(A) Einbringen, Ausrichten und Bündeln einer Probe (1), umfassend eine Vielzahl von Haaren, Fasern oder dünnen, flexiblen Materialien in einem zylindrisch ausgebildeten Innenraum mindestens eines Halteelements (2),
(B') Vorschieben der Probe (1) innerhalb des zylindrischen Innenraums unter Verwendung eines Stößels (10) und
(C') Schneiden der Probe (1) mit einer Schneidevorrichtung (3) vor dem Halteelement (2) unter Herstellung eines Schnittpräparates, wobei das Schnittpräparat eine Vielzahl von Abschnitten von Haaren, Fasern oder dünnen, flexiblen Materialien (12) umfasst.

6. Verwendung des Verfahrens gemäß einem der Ansprüche 1 bis 5 zur Herstellung von Haarpartikeln definierter Länge zur segmentalen Haaranalyse.

7. Verwendung des Verfahrens gemäß einem der Ansprüche 1 bis 5 zur Herstellung von Referenzproben zur Haaranalytik.

## Claims

1. A method for cutting hairs, fibers, or thin flexible materials comprising the following consecutive steps:
(A) introducing, aligning and bundling a sample (1), comprising a plurality of hairs, fibers, or thin flexible materials, in a cylindrically shaped interior of at least one holding member (2), and
(B) cutting the sample (1) and the holding member (2) with a cutting device (3) for obtaining a section (11),
wherein the section comprises a plurality of segments of hairs, fibers or thin, flexible materials (12) and a segment of the holding member (13).

2. Method according to claim 1, additionally comprising, subsequent to step (B), the following further step:
(C) collecting the section (11) in manner free from contamination.

3. Method according to claim 2, additionally comprising, subsequent to step (C), the following further step:
(D) separating the segment of the holding member (13) and the segments of hair, fibers, or thin flexible materials (12) for producing segment particles of a defined length.

4. Method according to any one of the claims 1 to 3 for producing a section (11) comprising a plurality of segments of hair, fibers, or thin flexible materials (12) and a segment of the holding member (13), wherein, after step (B) or during step (C), respectively, the plurality of segments of hair, fibers, or thin flexible materials remain connected detachably to the segment of the holding member (13).

5. A method for cutting hair, fibers, or thin flexible materials, comprising the following consecutive steps:
(A') introducing, aligning and bundling a sample (1), comprising a plurality of hairs, fibers, or thin flexible materials in a cylindrically shaped interior of at least one holding member (2),
(B') advancing the sample (1) within the cylindrical interior using a plunger (10) and
(C') cutting the sample (1) with cutting device (3) in front of the holding member (2) to produce a section, wherein the section comprises a plurality of segments of hair, fibers, or thin flexible materials (12).

6. Use of the method according to any one of the claims 1 to 5 for the production of hair particles of a defined length for use in segmental hair analysis.

7. Use of the method according to any one of the claims 1 to 5 for the preparation of reference samples for use in hair analysis.

## Revendications

1. Procédé de coupe de cheveux, de fibres ou de matériaux minces et flexibles, comprenant les étapes successives suivantes :
(A) mise en place, alignement et mise en faisceau d'un échantillon (1) comprenant une pluralité de cheveux, de fibres ou de matériaux minces et flexibles dans un espace intérieur de forme cylindrique d'au moins un élément de maintien (2), et
(B) sectionnement de l'échantillon (1) et de l'élément de maintien (2) par un dispositif de coupe (3) pour l'obtention d'une préparation de coupe (11), ladite préparation de coupe comprenant une pluralité de tronçons de cheveux, de fibres (12) ou de matériaux minces et flexibles et un tronçon de l'élément de maintien (13).

2. Procédé selon la revendication 1, comprenant en outre l'autre étape suivante consécutive à l'étape (B) :
(C) recueil exempt de contamination de la préparation de coupe (11).

3. Procédé selon la revendication 2, comprenant en outre l'autre étape suivante consécutive à l'étape (C) :
(D) séparation du tronçon de l'élément de maintien (13) et des tronçons de cheveux, de fibres ou de matériaux minces et flexibles (12) en obtenant des particules de coupe de longueur définie.

4. Procédé selon l'une des revendications 1 à 3 pour l'obtention d'une réparation de coupe (11), comprenant une pluralité de tronçons de cheveux, de fibres ou de matériaux minces et flexibles (12) et un tronçon de l'élément de maintien (13), la pluralité de tronçons de cheveux, de fibres ou de matériaux minces et flexibles restant fixée de manière détachable dans le tronçon de l'élément de maintien (13) après l'étape (B) ou pendant l'étape (C).

5. Procédé de coupe de cheveux, de fibres ou de matériaux minces et flexibles, comprenant les étapes successives suivantes :
(A) mise en place, alignement et mise en faisceau d'un échantillon (1) comprenant une pluralité de cheveux, de fibres ou de matériaux minces et flexibles dans un espace intérieur de forme cylindrique d'au moins un élément de maintien (2),
(B') refoulement de l'échantillon (1) dans l'espace intérieur cylindrique au moyen d'un poinçon (10) et
(C') sectionnement de l'échantillon (1) par un dispositif de coupe (3) en avant de l'élément de maintien (2) pour l'obtention d'une préparation de coupe, ladite préparation de coupe comprenant une pluralité de tronçons de cheveux, de fibres ou de matériaux minces et flexibles (12).

6. Utilisation du procédé selon l'une des revendications 1 à 5 pour l'obtention de particules de cheveux de longueur définie pour une analyse segmentaire des cheveux.

7. Utilisation du procédé selon l'une des revendications 1 à 5 pour l'obtention d'échantillons de référence pour une analyse des cheveux.
